(19) Europäisches Patentamt
European Patent Office
Office européen des brevets

(11) **EP 4 381 322 B1**

(12) **EUROPEAN PATENT SPECIFICATION**

(45) Date of publication and mention
of the grant of the patent:
**22.04.2026 Bulletin 2026/17**

(21) Application number: **22853854.2**

(22) Date of filing: **03.08.2022**

(51) International Patent Classification (IPC):
$G01S\ 15/89^{(2006.01)}$ $\quad G01N\ 29/07^{(2006.01)}$
$A61B\ 8/00^{(2006.01)}$

(52) Cooperative Patent Classification (CPC):
**A61B 8/08; A61B 8/5207; G01S 7/52036;
G01S 15/8977**

(86) International application number:
**PCT/US2022/039282**

(87) International publication number:
**WO 2023/014796 (09.02.2023 Gazette 2023/06)**

(54) **INVERSION OF SOUND SPEED AND POISSON'S RATIO FROM ULTRASOUND BEAM DATA FOR CHARACTERIZATION OF TISSUES**

INVERSION VON SCHALLGESCHWINDIGKEIT UND POISSONZAHL AUS ULTRASCHALLSTRAHLDATEN ZUR CHARAKTERISIERUNG VON GEWEBEN

INVERSION DE VITESSE DU SON ET DE COEFFICIENT DE POISSON À PARTIR DE DONNÉES DE FAISCEAU ULTRASONORE POUR CARACTÉRISATION DE TISSUS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.08.2021 US 202163229246 P**

(43) Date of publication of application:
**12.06.2024 Bulletin 2024/24**

(73) Proprietor: **Cloudstream Medical Imaging, Inc.
Houston, Texas 77242 (US)**

(72) Inventor: **PENG, Chengbin
Houston, Texas 77242 (US)**

(74) Representative: **Willett, Christopher David
Nash Matthews LLP
9 Hills Road
Cambridge CB2 1GE (GB)**

(56) References cited:
**US-A1- 2007 203 673      US-A1- 2009 112 093
US-A1- 2018 125 451      US-A1- 2020 124 752
US-A1- 2021 177 380**

• **ISLAM MD. TAUHIDUL ET AL: "Non-invasive
imaging of Young's modulus and Poisson's ratio
in cancers in vivo", vol. 10, no. 1, 29 April 2020
(2020-04-29), US, XP093274526, ISSN: 2045-2322,
Retrieved from the Internet <URL:https://www.
nature.com/articles/s41598-020-64162-6>
[retrieved on 20250505], DOI: 10.1038/
s41598-020-64162-6**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

This application claims priority to US Provisional Patent Application No. 63/229,246, filed on August 4, 2021.

**FIELD OF THE INVENTION**

The present invention relates to a method for obtaining a sound speed image and a Poisson's ratio image for characterization of the tissues.

**BACKGROUND OF THE INVENTION**

An ultrasound scanner produces pictures of the inside of human body using sound waves. It uses a small probe called a transducer placed directly on the skin. Highfrequency sound waves travel from the probe into the body. The probe collects sound reflections that bounce back from acoustic contrasts in tissues and organs. Ultrasound signals contain information about mechanical and acoustic properties of tissues and organs through which the ultrasound waves propagate or from which ultrasound waves are reflected. Those sound waves are used to create an image. Ultrasound imaging is a noninvasive medical test that helps a physician to diagnose and treat medical conditions [1, 2].

Medical ultrasound applications need to make a step change from general diagnostic imaging to quantitative characterization of tissues and organs. Seeing a feature on an ultrasound image is one thing. Knowing what is inside the feature is another thing. The latter ought to be more useful for clinical applications. In this way we can make ultrasound imaging technologies more competitive with or complementary to X-CT and MRI modalities. Attempts were made to measure physical properties of tissues using ultrasound. Shear wave elastography was an example. It uses shear wave fronts excited by a moving focused ultrasound beam to measure shear wave speeds in tissues [3, 4]. Travel time tomography was also used to invert for compressional wave velocities in tissues. It uses the travel time of sound waves between a pair of transmitter and receiver [5, 6]. Diffraction tomography was used for breast imaging. It uses diffracted waves to invert for compressional wave speed distribution in breast tissues [7]. Recently deep learning and artificial intelligence methods were used to invert for sound speed in medical ultrasound, using computer generated synthetic ultrasound data as training dataset since a large amount of training datasets is hard to come by [8]. Full waveform inversion was also reported in literature where a supercomputer was used to invert for sound speed variations by exactly matching recorded waveforms with computer generated waveforms [9, 10].

Commercial ultrasound scanners are good at producing a diagnostic image to show shapes and structures of tissues and organs under examination. The images could not tell other important properties that are needed to characterize the health of the tissues, such as, hardness of the tissues, density, sound speed, Poisson's ratio, mechanical moduli, water content, to name a few. Clinic physicians want to know more about features and lesions seen on an ultrasound image.

**SUMMARY OF THE INVENTION**

The invention is set out in the appended set of claims. The present invention relates to ultrasound imaging in general. In particular, the invention extends ultrasound to detailed characterization of physical properties of tissues and organs. In one way we can obtain not only a reflectivity image (B-mode image) but also the sound speed value at every image point at the same time. The B-mode reflectivity image and sound speed image together can be used to perform more detailed characterization of tissues and organs under examination. B-Mode is a two-dimensional ultrasound image display composed of bright dots representing ultrasound echoes with brightness proportional to echo strength.

In another way we can obtain not only a reflectivity image but also Poisson's ratio value at every image point at the same time. Poisson's ratio is an important material property. The value of Poisson's ratio is 0.5 for water, or slightly less than 0.5 for soft tissues that contain a lot of water in content. Muscular tissues have Poisson's ratio in the range of 0.30 - 0.45. Bones have Poisson's ratio values in the range of 0.35 - 0.4. Kidney stones have even smaller Poisson's ratio values (< 0.3). The B-mode reflectivity image and the Poisson's ratio image together can be used to perform more detailed characterization of tissues and organs under examination.

In one embodiment, the present application discloses a method for obtaining a sound speed image, and the method includes the steps of: (1) spraying, with a processor, a data sample of an ultrasound beam, measured with an apparatus that contains an ultrasound array transducer, into an image space of a subject along an impulse response curve; (2) collecting, with the processor, contributions of the data sample in the image space; (3) summing contributions of a plurality of data samples by repeating the step (1) and the step (2) for a plurality of ultrasound beams, with the processer, to generate a plurality of partial images, and storing the partial images in a first memory location; (4) sorting, with the processor, the partial images to form common image point gathers at a plurality of spatial locations, and storing the common image point gathers in a second memory location; (5) measuring, with the processor, residual moveout values on the common image point gathers, each residual moveout value relating to a corresponding spatial location; and (6) inverting, with the processor, the residual moveout values to form an image of the sound speed, and sending the sound speed image to a net address via TCP/IP, a display port on a host computer, or a third memory location.

In another embodiment, the step (6) includes: setting an initial sound speed; and calculating, with the processor, an updated sound speed value at the corresponding spatial location using the initial sound speed and the each residual moveout value.

In another embodiment, the initial sound speed is 1,540 m/s.

In another embodiment, the step (6) further includes: applying a spatial smoothing, with the processor, to the sound speed image to remove rapid variations.

In another embodiment, the present application provides a method for obtaining a Poisson's ratio image, and the method includes the steps of: (1) spraying, with a processor, a data sample of an ultrasound beam, measured with an apparatus that contains an ultrasound array transducer, into an image space of a subject along an impulse response curve; (2) collecting, with the processor, contributions of the data sample in the image space; (3) summing the contributions of a plurality of data samples by repeating the step (1) and the step (2) for a plurality of ultrasound beams, with the processer, to generate a plurality of partial images, and storing the partial images in a first memory location; (4) sorting, with the processor, the partial images to form common image point gathers at a plurality of spatial locations, and storing the common image point gathers in a second memory location; (5) measuring, with the processor, residual moveout values on the common image point gathers, each residual moveout value relating to a corresponding spatial location; (6) applying, with the processor, residual moveout corrections to flatten the common image point gathers; (7) measuring, with the processor, amplitudes and reflection angles on the common image point gathers, each pair of amplitude and reflection angle relating to a corresponding spatial location; and (8) inverting, with the processor, the amplitudes and reflection angles to form a Poisson's ratio image, and sending the Poisson's ratio image to a net address via TCP/IP, a display port on a host computer, or a third memory location.

In another embodiment, the step (8) comprises: estimating a normal incident reflection amplitude; and calculating, with the processor, a proxy of Poisson's ratio at the corresponding spatial location using the normal incident reflection amplitude and the measured amplitudes at a plurality of reflection angles.

In another embodiment, the step (8) comprises: estimating a normal incident reflection amplitude; and calculating, with the processor, a proxy of shear wave speed contrast at the corresponding spatial location using the normal incident reflection amplitude and the measured amplitudes at a plurality of reflection angles.

It is to be understood that both the foregoing general description and the following detailed description are exemplary and explanatory and are intended to provide further explanation of the invention as claimed.

US2021177380A1 discloses a method and apparatus for quantitative ultrasound imaging using single ultraouns probe.

ISLAM MD. TAUHIDUL ET AL: "Non-invasive imaging of Young's modulus and Poisson's ratio in cancers in vivo", SCIENTIFIC REPORTS, vol. 10, no. 1, 29 April 2020, US ISSN: 2045-2322, DOI: 10.1038/s41598-020-64162-6, discloses non-invasive imaging of Young's modulus and Poisson' s ratio in cancers in vivo.

## BRIEF DESCRIPTION OF THE DRAWINGS

The accompanying drawings, which are included to provide a further understanding of the invention and are incorporated in and constitute a part of this specification, illustrate embodiments of the invention and together with the description serve to explain the principles of the invention.

In the drawings:

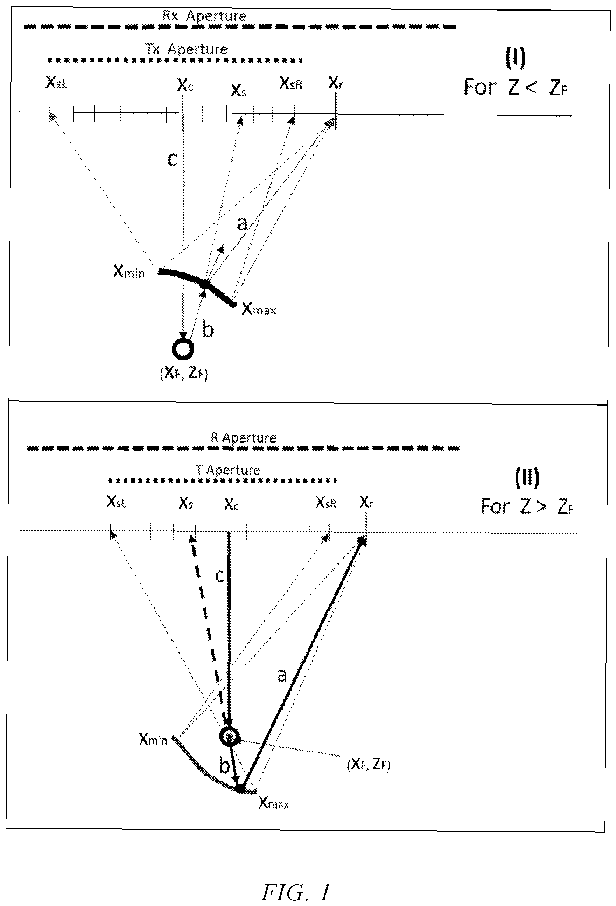

Figure 1 shows finite support $[X_{min}, X_{max}]$ of a Beam Impulse Response Curve. An impulse response of a beamforming operator is a curve (thick line) that contains all possible image points that one sample in a beam record contributes to. The open circle is the focus for this beam with a $T_x$ aperture (of active transmitters) and a $R_x$ aperture (of active receivers). The solid black dot at (x, z) is an output image point. Here $X_c$ is the beam center coordinate. $X_r$ is the receiver coordinate for this data sample. $X_s$ is the stationary phase solution of the effective transmitter coordinate. Offset H is the distance between $X_r$ and $X_s$. Reflection angle is half of the intersection angle between a line from $X_s$ to (x, z) and another line from $X_r$ to (x, z). Top (I) is for the case $Z < Z_F$. Bottom (II) is for the case $Z > Z_F$.

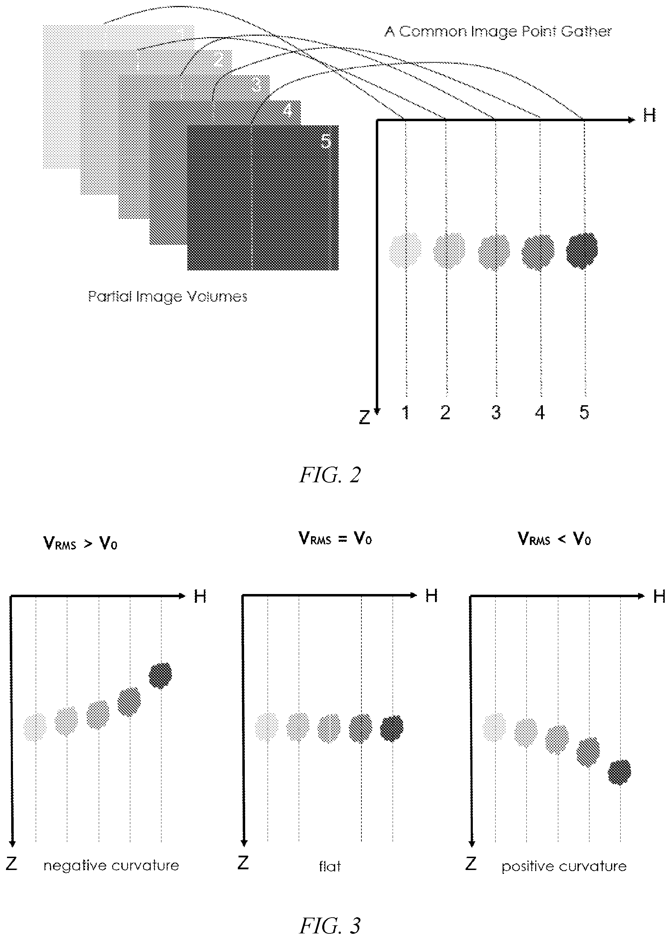

Figure 2 shows the formation of a common image point gather: at each fixed lateral position we extract one vertical trace from each partial image volume and put them side by side. This organization of beamformed partial images is called a common image point gather. The vertical axis is image depth or two-way vertical time. The horizontal axis is the binning attribute used in formation of the partial images. We use the index number of each partial image volume in this illustration. We can also use the physical value of the binning attribute to label the horizontal axis.

Figure 3 shows residual moveout curvatures on common image point gathers: the left panel has negative curvature, indicating $V_0 < V_{RMS}$; the middle panel has zero curvature, indicating $V_0 = V_{RMS}$; the right panel has positive curvature,

indicating $V_0 > V_{RMS}$.

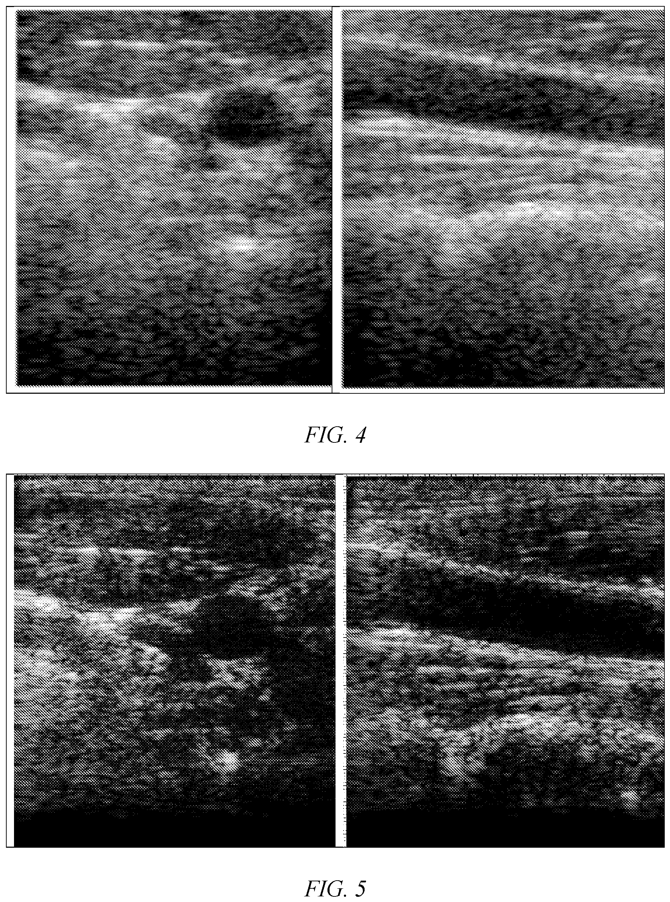

Figure 4 shows two published B-mode images on USTB website (www.ustb.no) of PICMUS carotid challenge dataset: (left) a transversal image of neck artery, and (right) a longitudinal image of the same. All displays are in 60 dB with log compression, with white color representing bright (high) amplitudes and black color for dim (low) amplitudes.

Figure 5 shows two B-mode images obtained with our method using the PICMUS carotid challenge dataset as input: (left) a transversal image of neck artery, and (right) a longitudinal image of the same. All displays are in 60 dB with log compression, with white color representing bright (high) amplitudes and black color for dim (low) amplitudes.

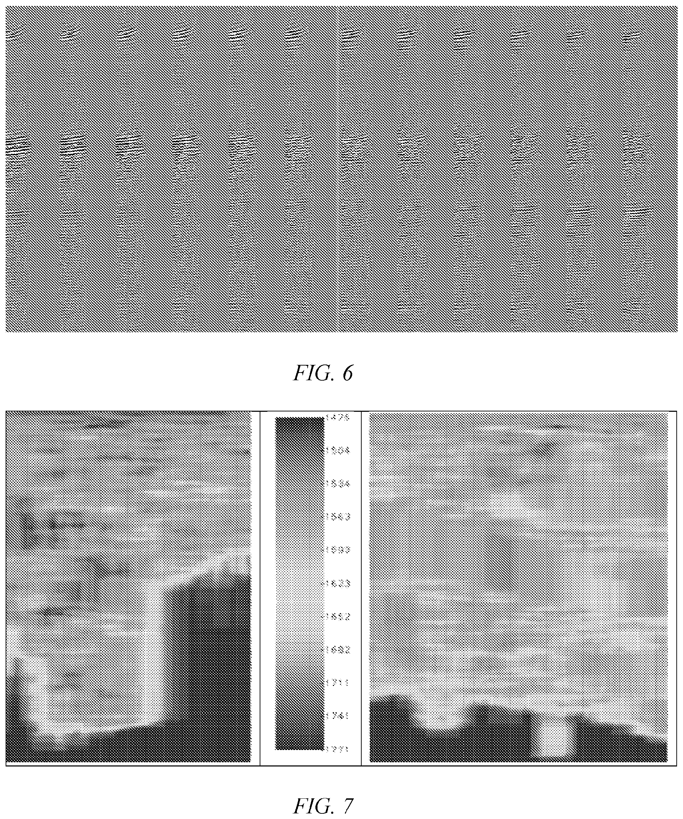

Figure 6 show common image point gathers obtained with our method using the PICMUS carotid challenge dataset. Each panel shows a common image point gather at a given lateral location. Within each gather the vertical axis is depth and the horizontal axis is transmitter-receiver offset.

Figure 7 shows a sound speed image using the PICMUS carotid challenge dataset: (left) a transversal view in sound speed of neck artery, and (right) a longitudinal view of the same. The color bar is in m/s.

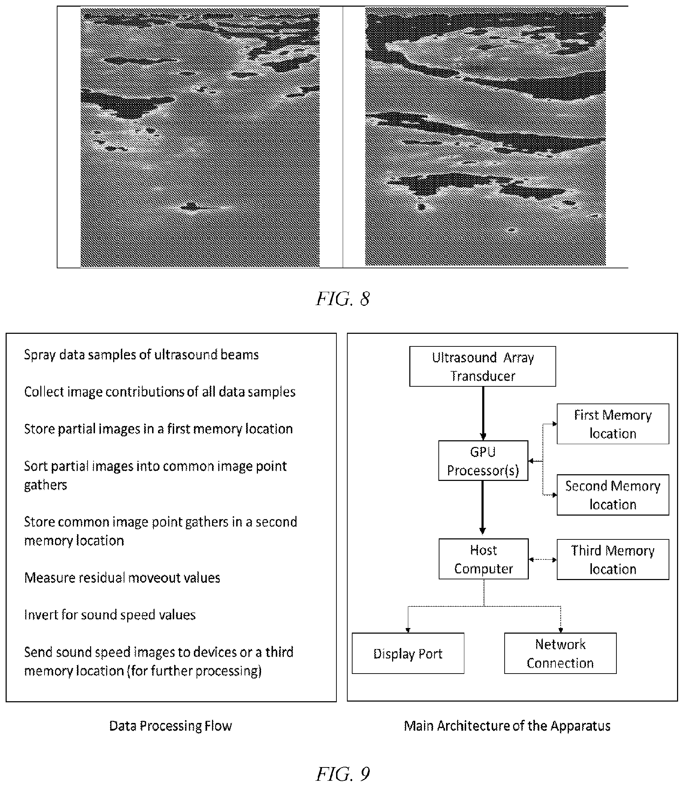

Figure 8 shows a Poisson's ratio (proxy) image using the PICMUS carotid challenge dataset: (left) a transversal view of neck artery, and (right) a longitudinal view of the same.

Figure 9 shows main components of an apparatus for imaging sound speed property of tissues, and the apparatus includes an ultrasound array transducer that transmits and receives echo data and one or more GPU processors that (1) sprays data samples of ultrasound beams into the image space along their impulse response curves, (2) collects image contributions of all data samples to generate a plurality of partial images, (3) stores the partial images in a first memory location, (4) sorts the partial images into a plurality of common image point gathers, (5) stores the common image point gathers in a second memory location, (6) measures residual moveout values at a plurality of spatial locations, and (7) inverts the residual moveout values to obtain a sound speed image. The apparatus also includes a host computer that sends the sound speed image to a net address via TCP/IP, or a display port on the host computer, or a third memory location for further processing.

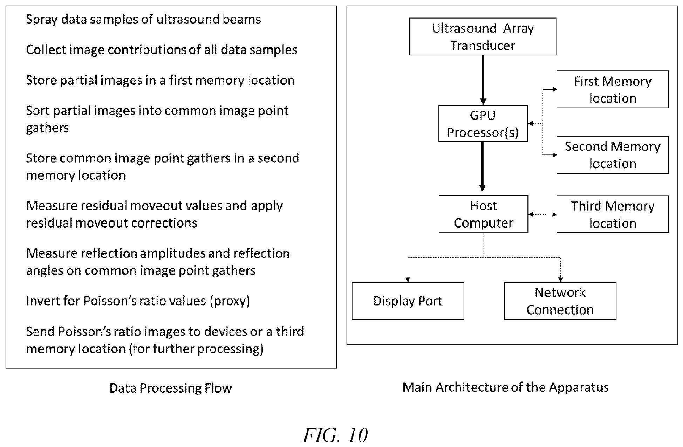

Figure 10 shows main components of an apparatus for imaging Poisson's ratio (proxy) property of tissues, and the apparatus includes an ultrasound array transducer that transmits and receives echo data and one or more GPU processors that (1) sprays a data sample of an ultrasound beam into the image space along its impulse response, (2) collects image contributions of all data samples to generate a plurality of partial images, (3) stores the partial images in a first memory location, (4) sorts the partial images into a plurality of common image point gathers, (5) stores the common image point gathers in a second memory location, (6) measures residual moveout values at a plurality of spatial locations, (7) applies residual moveout corrections on common image point gathers, (8) measures reflection amplitudes and reflection angles at a plurality of spatial locations, and (9) inverts the pairs of amplitude and reflection angle data to obtain a Poisson's ratio (proxy) image. The apparatus also includes a host computer that sends the Poisson's ratio (proxy) image to a net address via TCP/IP, or a display port on the host computer, or a third memory location for further processing.

## DETAILED DESCRIPTION OF THE ILLUSTRATED EMBODIMENTS

Reference will now be made in detail to embodiments of the present invention, example of which is illustrated in the accompanying drawings.

Traditional beamforming of ultrasound data utilizes dynamic focusing method implemented on FPGA hardware [1, 2]. Modern ultrasound imaging applications use pixel-based beamforming methods [11 - 16], mostly implemented on GPU hardware. All these methods can be easily understood by examining spatial impulse responses of the beamforming operators applied on individual data sample collected by an ultrasound scanner. An impulse response of a beamforming operator, by our definition, is a curve in image domain with a finite support. It contains all possible image points that one data sample in one input ultrasound beam contributes to. The final image is formed by summing all impulse responses for all data samples of all input beams. Figure 1 depicts one impulse response for a focused beam (thick black curve). Similar impulse responses can be produced for a divergent beam and a planewave beam. In Figure 1 an acquisition layout for one input beam is shown. Active transmitters are covered by the short dash line labeled Tx aperture. Active receivers are covered by the long dash line labeled Rx aperture. These two apertures can be the same or different without any impact on our analysis herein. The leftmost transmitter is at $X_{sL}$ and the rightmost transmitter is at $X_{sR}$. The beam center is at $X_c$. The focal point is at $(X_F, Z_F)$. One time sample recorded by a receiver at $X_r$ is used to produce the impulse response curve. The impulse response curve has a finite support between $[X_{min}, X_{max}]$ beyond which there are no stationary reflections for this data sample. This physical restriction is ignored by all published pixel-based beamforming methods. It is also worthwhile to note that the traditional dynamic focusing method corresponds to a single point on the impulse response curve at the location where the curve intersects with the scanline of this beam. In this regard the dynamic focusing method is wasteful in utilizing the ultrasound beam data for imaging because only one point is imaged by a whole beam.

Formulas are given in the box in Figure 1 for the calculation of the impulse response and its spatial support. $X_s$ is the stationary location of a transmitter that makes a significant contribution to the image at the output point $(x, z)$. The

distance between $X_s$ and $X_r$ is called the stationary offset, or simply offset. The reflection angle at (x, z) is called the stationary specular reflection angle, or simply reflection angle. Each point on the impulse response curve carries distinct values of offset, reflection angle, dip angle, to name a few. Partial image volumes can be generated by binning and sorting each point on the impulse response curve according to the value of offset, reflection angle, or some other attribute. In the box, ta is the travel time from the receiver to the image point, tb is the travel time from the focal point to the image point, and tc is the travel time from the focal point to the beam center. The arrival time of the input data sample is t.

A general formulation of impulse responses for ultrasound beam data of arbitrary types can be found in our separate patent application, US. Provisional Patent Application No. 63/184174, filed May 4, 2021, published as US 2024/210560 A1.

A common image point gather is formed by sorting all partial images at a fixed output location into a gather as shown in Figure 2. A partial image volume is generated, during beamforming, by binning and sorting each point on an impulse response curve according to the value of offset, reflection angle, or some other attribute. The final image is formed by stacking common image point gathers at every output location.

It is worthwhile to note that, if the correct sound speed is used in beamforming, the common image point gather in Figure 2 should be flat. These partial images will coherently compound together to form an enhanced image with good resolution and good signal to noise ratio. A detailed description of common image point gathers of ultrasound beam data can be found in our separate patent application, US Provisional Patent Application No. 63/197932, Filed Jun 7, 2021, published as US 2024/206854 A1.

**Inversion for Spatially Varying Sound Speed**

Residual Moveout Analysis

The best sound speed value that yields a focused image of ultrasound data at a given spatial location is the RMS (root-mean-square) equivalent of the true sound speed distribution in tissues that acoustic waves travel through. Sound speed varies spatially in tissues, either continuously or in a discontinuous fashion. The RMS equivalent of sound speed, at a given location, is given by:

$$V_{RMS}{}^2(T) = \frac{1}{T}\int_0^T V^2(t)\, dt \tag{1}$$

where V(t) is a sound speed profile that is converted into vertical two-way time.

If the sound speed value used in beamforming ($V_0$) is equal to the effective sound speed value ($V_{RMS}$) in equation (1), then all partial images should be spatially co-located (i.e., the same tissue structure is imaged multiple times from different illuminations). In this case one should see a flat common image point gather for reflection data at every output location (Figure 3, middle). Non-flat common image point gathers indicate that the sound speed value used in beamforming is not optimal for reflection data, either a wrong value is chosen or the actual sound speed in tissues is spatially varying such that a constant value could not represent the true sound speed variation inside a human body. In the case $V_0 < V_{RMS}$ residual moveout curvature is negative (Figure 3, left). In the case $V_0 > V_{RMS}$ residual moveout curvature is positive (Figure 3, right). Therefore, residual moveout curvatures on common image point gathers can be used to invert for sound speed distribution in the image domain.

The correct sound speed value $V_1$ (RMS equivalent) that yields the best focused images at a given spatial location is related to the residual moveout curvature according to the following formula:

$$\frac{1}{V_1{}^2} = \frac{1}{V_0{}^2} + \frac{1}{V_{RMO}{}^2} \tag{2}$$

where $V_0$ is the sound speed used in an initial beamforming (typically 1540 m/s). $V_{RMO}$ is the inverse of the residual moveout curvature we measure on a common image point gather. If the gather is flat, i.e., the curvature term is zero, the second term in the right-hand side of equation (2) vanishes. This leads to $V_1 = V_0$. If the residual moveout curvature is positive, we will have $V_1 < V_0$. If the residual moveout curvature is negative, we will have $V_1 > V_0$.

**The Dix Inversion**

The Dix inversion formula has not been used in B-mode ultrasound imaging for medical applications. The inversion formula proposed by Dix [19] is given as:

$$V^2(T_1, T_2) = \frac{T_2\, V_{RMS}{}^2(T_2) - T_1\, V_{RMS}{}^2(T_1)}{T_2 - T_1} \tag{3}$$

where $V(T_1, T_2)$ is the estimated average sound speed between two-way vertical time $T_1$ and $T_2$. Inputs to the Dix inversion are RMS equivalent sound speeds at the time samples.

The Dix inversion is one dimensional in nature and its result looks blocky between data points. Editing out some outliers and applying some spatial smoothing are common practices in geophysical applications [20].

**Inversion for Spatially Varying Poisson's Ratio**

The use of amplitude information (or echo strength) in B-mode ultrasound imaging is mostly for identification of echoic regions and anechoic regions in human bodies. We extend the use of amplitude measurements on common image point gathers to directly invert or infer physical property distributions in tissues, such as Poisson ratio, Young's moduli, compressional impedance, shear impedance, density, water content, to name a few. The data used in our inversion or inference are pairs of amplitudes and reflection angles (or offsets) measured on common image point gathers [21, 22]. The methodology is not known in medical applications such as B-mode ultrasound imaging.

If contrasts in acoustic properties across an internal boundary are small, the reflection amplitudes vary with the reflection angles according to the following formula:

$$R(\theta) = A + B\,\sin^2\theta \tag{4}$$

where A is the normal incidence reflection amplitude and B is the gradient in a cross plot of amplitude $R(\theta)$ and $\sin^2\theta$. In a linearized approximation they are related to contrasts in physical parameters as follows [23, 24]:

$$A = \frac{1}{2}\left(\frac{\Delta V}{V} + \frac{\Delta\rho}{\rho}\right) \tag{5a}$$

$$B = -\frac{3-7\sigma}{2(1-\sigma)}\frac{\Delta V}{V} - \frac{1-2\sigma}{(1-\sigma)}\frac{\Delta\rho}{\rho} + \frac{1}{(1-\sigma)^2}\Delta\sigma \tag{5b}$$

where V is sound speed (compressional wave), $\rho$ is density, and $\sigma$ is Poisson's ratio. $\frac{\Delta V}{V}$ is the relative change of sound speed across an internal boundary. $\frac{\Delta\rho}{\rho}$ is the relative change of density. $\Delta\sigma$ is the absolute change of Poisson's ratio.

Human tissues are 90% or more water in content. Values of Poisson's ratio in human tissues are close to 0.5 which is the Poisson's ratio of water. In this case the first two terms of B in equation (2b) are much smaller than the third term. The gradient B is mostly related to the contrast in Poisson's ratio $\sigma$ for medical ultrasound applications. We shall call the gradient B a proxy of Poisson's ratio in these cases. In all examples below we are using the gradient B value as a proxy of Poisson's ratio attribute in our analysis.

In human tissues the relative change of compressional wave speed is much smaller than the relative change of shear wave speed, i.e., $\left|\frac{\Delta V}{V}\right| \ll \left|\frac{\Delta V_S}{V_S}\right|$. It is easy to show that:

$$\frac{\Delta V_S}{V_S} = -\frac{1}{2(1-\sigma)(1-2\sigma)}\Delta\sigma \tag{6}$$

Therefore, the gradient B is also negatively correlated with the relative change of shear wave speed:

$$B = -\frac{2(1-2\sigma)}{(1-\sigma)}\frac{\Delta V_S}{V_S} \tag{7}$$

The significance of equation (7) is that we can obtain shear wave information using B-mode ultrasound beam data without actual excitation of shear waves in tissues, which is different from shear wave elastography [3, 4].

The Poisson's ratio proxy (also shear wave reflectivity proxy) can be obtained from a parametric fitting / linear regression of equation (4) on a common image point gather on which both reflection amplitudes and reflection angles are measured. We first compute envelopes of traces on the common image point gather in order to remove the impact of wavelet polarity on the regression or fitting. We then perform the two parameters (A and B) regression using the envelope amplitudes. The resulting B value is a good indication how amplitudes vary with reflection angles: a positive B value corresponds to the case of the envelope amplitude increasing with reflection angle; a negative B value corresponds to the case of the envelope amplitude decreasing with reflection angle. We repeat this process for all output locations to obtain an image of the Poisson's ratio proxy (i.e., B value). The image can also be interpreted as a proxy of the shear wave reflectivity.

Positive B values are typically associated with soft tissues with a lot of water content ($\sigma$ *is close to* 0.5). Negative B values are typically associated with harder or solid tissues with less water content ($\sigma < 0.45$). The B value (Poisson's ratio proxy) is sensitive to water content, which shares similarity with MRI images used by clinical physicians. It is also sensitive to calcification of tissues (such as liver) where the values of Poisson's ratio decrease and the values of shear wave speed increase significantly.

### *In Vivo* Carotid Ultrasound Data Example

PICMUS Carotid Challenge Data

PICMUS is the IEEE US 2016 Plane-wave Imaging Challenge in Medical UltraSound, an initiative of IEEE US to promote use of plane-wave ultrasound imaging modality. PICMUS carotid challenge dataset is a public domain dataset for download at Ultrasound Test Benchmark (USTB) website (https://www.ustb.no/ustb-datasets). The dataset contains two in vivo carotid scans, one in the cross section and another in longitudinal section, collected by a volunteer with a Verasonics Vantage 256 System and a Verasonics L11 transducer. The use of this dataset is subject to citation rule (https://www.ustb. no/examples/picmus/picmus-invivo-carotid-cross and https://www.ustb.no/examples/picmus/picmus-invivo-carotid-long). We sincerely thank IEEE US for making this dataset available in the public domain.

The images in Figure 5 are published B-mode images on USTB website for benchmark purposes. The raw ultrasound data is collected in two directions, one measurement (left) is in a direction transversal to the neck artery in-vivo, and the other measurement (right) is in a longitudinal direction. Bright colors represent stronger acoustic echoes when an ultrasound beam is reflected from tissue contrasts in acoustic impedance. Darker regions are anechoic, with much smaller acoustic contrasts. Public are encouraged to produce better results with improved algorithms.

Enhanced B-Mode Images

We are able to produce improved images with the method disclosed in this invention, using the same PIMCUS carotid raw ultrasound data as input. Figure 6 shows our results: improvements can be seen in both contrast resolution and spatial resolution, with clear definition of walls of carotid artery (circular features on the left and elongated features on the right), showing no build-up of fatty deposits on the vessel walls. The dark regions are either anechoic or acoustic waves being strongly attenuated during wave propagation (e.g., near the bottom).

Sound Speed Images

To obtain a spatially varying sound speed image we first generate a set of common image point gathers using the PICMUS carotid challenge dataset. Since sound waves in different tissues propagate with different speed values, a beamforming algorithm that uses a constant value for sound speed will always produce non-flat common image point gathers as shown in Figure 7. The residual moveout on the common image point gathers can be accurately measured. The Dix inversion is then applied to obtain sound speed distribution in the image domain. Figure 7 shows resulting sound speed images: the left one is a sound speed image in the transversal direction, and the right one is another sound speed image in the longitudinal direction. One can see lower sound speed values inside the carotid vessels. Soft tissues also have lower sound speed values. Muscle tissues have higher sound speed values. Since the Dix inversion is a 1-D method we typically see vertical stripes in the output. One can apply a spatial smoothing to remove the vertical stripes, if desired.

It is worthwhile to note that the estimated values of sound speed at the bottom part of the images are not reliable as acoustic waves are strongly attenuated by tissue absorption before they can reach the transducer placed on the top surface (dark color in Figure 7). We can cut out the deep portion, if desired. Figure 9 is a schematic diagram of the method and the apparatus for obtaining a sound speed image.

Poisson's Ratio Images

To obtain an image of spatially varying Poisson's ratio we generate a set of common image point gathers using the PICMUS carotid challenge dataset. We measure the residual moveouts on the common image point gathers. We perform a residual moveout correction to flatten the common image point gathers. We measure amplitudes on the common image point gathers and estimate B values using the amplitude vs. sine squared reflection angle cross-plot. The B value serves as a proxy of Poisson's ratio (also a good proxy of shear wave reflectivity). Fluid and fatty tissues have larger Poisson's ratio value. Muscle tissues and bones have smaller Poisson's ratio value. Therefore, we can use the Poisson's ratio value to aid our characterization of various tissues. Figure 8 shows resulting Poisson's ratio (proxy) images: the left one is an image of Poisson's ratio (proxy) in the transversal direction, and the right one is another image in the longitudinal direction. One can see higher Poisson's ratio values (light gray color) inside the carotid artery (fluid) and soft tissues. In muscular tissues the Poisson's ratio values are smaller (dark region). The Poisson's ratio (proxy) image is useful in delineating body fluids and fatty tissues. Figure 10 is a schematic diagram of the method and the apparatus for obtaining a Poisson's ratio image.

References

[1] Richard S. C. Cobbold (2007), Foundations of Biomedical Ultrasound, Oxford University Press, pages 1416-1428.

[2] F. W. Kremkau (2006), Diagnostic Ultrasound: Principles and Instruments, 7th edition, Saunders/ Elsevier, St. Louis.

[3] J. Ophir, S. K. Alam, B. S. Garra, F. Kallel, E. E. Knonfgou, T. Krousko, and C. R. B. Berritt (2002), Elastography: Imaging the Elastic Properties of Soft Tissues with Ultrasound, J. Medical Ultrasonics, Vol 29, pages 155 - 171.

[4] J. Bercoff, M. Tanter, T. M. Nguyen, J. M. Chassot, M. Fink, and C. Boccara (2004), Supersonic Shear Imaging: A New Technique for Soft Tissue Elasticity Mapping, IEEE Trans. On Ultrasonics, Ferroelectrics and Frequency Control, Vol 51, pages 396 - 409.

[5] M. Sak, N. Duric, P. Littrup, L. Bey-Knight, H. Ali, P. Vallieres, M. E. Sherman and G. L. Gierach (2017), Using Speed of Sound Imaging to Characterize Breast Density, Ultrasound in Medicine & Biology, Vol 43, pages 91 - 103.

[6] C. Li, A. Stewart and N. Duric (2012), Multi-grid Tomographic Inversion for Breast Ultrasound Imaging, Proceedings of SPIE, Vol 8320, pages 1-9.

[7] J. Nebeker and T. R. Nelson (2012), Imaging of Sound Speed Using Reflection Ultrasound Tomography, Journal of Ultrasound in Medicine, Vol 31, pages 1389 - 1404.

[8] Micha Feigin, D. Freedman, and B. W. Anthony, A Deep Learning Framework for Single-Sided Sound Speed Inversion in Medical Ultrasound, IEEE Transactions on Biomedical Engineering, Vol. 67, pages 1142- 1150.

[9] J. Wiskin, B. Malik, D. Borup, N. Pirshafiery and J. Klock (2020), Full Wave 3D Inverse Scattering Transmission Ultrasound Tomography in the Presence of High Contrast, Nature, https://doi.org/10.1038/s41498-020-76754-3.

[10] L. Guasch, O. Calderon-Agudo, M. X. Tang, P. Nachev, and M. Warner (2020), Full-waveform Inversion Imaging of the Human Brain, Nature, https://doi.org/10.1038/s41746-020-0240-8.

[11] R. E. Daigle (2009), Ultrasound Imaging System with Pixel Oriented Processing, U.S. Patent Application No. 2009/0112095 A1, April 30, 2009.

[12] R. Zemp and M. F. Insana (2007), Imaging with Unfocused Regions of Focused Ultrasound Beams, J. Acoust. So. Amer. Vol. 121, pages 1491-1498.

[13] N. Q. Nguyen and Richard Q. Prager (2016), High-resolution Ultrasound Imaging with Unified Pixel-Based Beamforming, IEEE Transactions on Medical Imaging, Vol. 35, pages 98-108.

[14] O. M. H. Rindal (2019), Software Beamforming in Medical Ultrasound Imaging - a Blessing and a Curse, Ph.D. Thesis, University of Oslo.

[15] O. M. H. Rindal, A. Rodriguez-Molares, and A. Austeng (2018), A Simple, Artifact-free, Virtual Source Model, IEEE International Ultrasonics Symposium, IUS 1-4. https://doi.org/10.1109/ultsym.2018.8579944.

[16] D. J. Napolitano, B. D. DeBusschere, G. W. McLaughlin, L. Y. Mo, C. H. Chou, T. L. Ji, R. W. Steins (2011), Continuous Transmit Focusing Method and Apparatus for Ultrasound Imaging Systems, US Patent 8,002,705, Issued Aug, 2011.

[17] C. Peng and J. Tang (2021), Acquisition and Processing of V-Wave Ultrasound Data Using a Linear or Curved Array Transducer, US. Patent Appl No. 63/184174, Filed May 4, 2021.

[18] C. Peng, and J. Tang (2021), Imaging Tissues and Organs Behind Bones Using an Ultrasound Array Transducer, US Patent Appl No. 63/197932, Filed Jun 7, 2021.

[19] C. H. Dix (1955), Seismic Velocities from Surface Measurements, Geophysics, Vol. 20, pages 68 - 86.

[20] Z. Koren and I. Ravve (2006), Constrained Dix Inversion, Geophysics, Vol. 71, pages R113 - R130.

[21] K, Aki and P. G. Richards (1980), Quantitative Seismology: Theory and Methods, W. H. Freeman and Co.

[22] O. Yilmaz (2011), Seismic Data Analysis: Processing, Inversion and Interpretation of Seismic Data, Society of Exploration Geophysicists.

[23] R. Bortfeld (1961), Approximation to the Reflection and Transmission Coefficients of Plane Longitudinal and Transverse Waves, Geophysical Prospecting, Vol 9, pages 485 - 503.

[24] J. P. Castagna, H. W. Swan, and D. J. Foster (1998), Framework for AVO Gradient and Intercept Interpretation, Geophysics, Vol 63, pages 948 - 956.


**Claims**

1. A method for obtaining a sound speed image, comprising the steps of:

   (1) spraying, with a processor, a data sample of an ultrasound beam, measured with an apparatus that contains an ultrasound array transducer, into an image space of a subject along an impulse response curve;
   (2) collecting, with the processor, contributions of the data sample in the image space;
   (3) summing contributions of a plurality of data samples by repeating the step (1) and the step (2) for a plurality of ultrasound beams, with the processer, to generate a plurality of partial images, and storing the partial images in a first memory location;
   (4) sorting, with the processor, the partial images to form common image point gathers at a plurality of spatial locations, and storing the common image point gathers in a second memory location;
   (5) measuring, with the processor, residual moveout values on the common image point gathers, each residual moveout value relating to a corresponding spatial location; and
   (6) inverting, with the processor, the residual moveout values to form an image of the sound speed, and sending the sound speed image to a net address via TCP/IP, a display port on a host computer, or a third memory location.

2. The method of claim 1, wherein the step (6) comprises:

   setting an initial sound speed; and
   calculating, with the processor, an updated sound speed value at the corresponding spatial location using the initial sound speed and the each residual moveout value.

3. The method of claim 2, wherein the initial sound speed is 1,540 m/s.

4. The method of claim 2, wherein the step (6) further comprises:
   applying a spatial smoothing, with the processor, to the sound speed image to remove rapid variations.

5. A method for obtaining a Poisson's ratio image, comprising the steps of:

   (1) spraying, with a processor, a data sample of an ultrasound beam, measured with an apparatus that contains an ultrasound array transducer, into an image space of a subject along an impulse response curve;
   (2) collecting, with the processor, contributions of the data sample in the image space;
   (3) summing the contributions of a plurality of data samples by repeating the step (1) and the step (2) for a plurality of ultrasound beams, with the processer, to generate a plurality of partial images, and storing the partial images in a first memory location;
   (4) sorting, with the processor, the partial images to form common image point gathers at a plurality of spatial locations, and storing the common image point gathers in a second memory location;
   (5) measuring, with the processor, residual moveout values on the common image point gathers, each residual moveout value relating to a corresponding spatial location;
   (6) applying, with the processor, residual moveout corrections to flatten the common image point gathers;
   (7) measuring, with the processor, amplitudes and reflection angles on the common image point gathers, each pair of amplitude and reflection angle relating to a corresponding spatial location; and
   (8) inverting, with the processor, the amplitudes and reflection angles to form a Poisson's ratio image, and sending the Poisson's ratio image to a net address via TCP/IP, a display port on a host computer, or a third memory location.

6. The method of claim 5, wherein the step (8) comprises:

   estimating a normal incident reflection amplitude; and
   calculating, with the processor, a proxy of Poisson's ratio at the corresponding spatial location using the normal incident reflection amplitude and the measured amplitudes at a plurality of reflection angles.

7. The method of claim 5, wherein the step (8) comprises:

estimating a normal incident reflection amplitude; and

calculating, with the processor, a proxy of shear wave speed contrast at the corresponding spatial location using the normal incident reflection amplitude and the measured amplitudes at a plurality of reflection angles.

**Patentansprüche**

1. Verfahren zum Erhalten eines Schallgeschwindigkeitsbildes, die Schritte umfassend:

   (1) Verteilen, mit einem Prozessor, einer Datenprobe eines Ultraschallstrahls, die mit einem Gerät gemessen wurde, das einen Ultraschall-Array-Wandler enthält, in einen Bildraum eines Subjekts entlang einer Impulsantwortkurve;
   (2) Sammeln, mit dem Prozessor, von Beiträgen der Datenprobe im Bildraum;
   (3) Summieren der Beiträge einer Vielzahl von Datenproben durch Wiederholen des Schritts (1) und des Schritts (2) für eine Vielzahl von Ultraschallstrahlen mit dem Prozessor, um eine Vielzahl von Teilbildern zu erzeugen, und Speichern der Teilbilder an einem ersten Speicherort;
   (4) Sortieren, mit dem Prozessor, der Teilbilder, um gemeinsame Bildpunktsammlungen an einer Vielzahl von räumlichen Orten zu bilden, und Speichern der gemeinsamen Bildpunktsammlungen an einem zweiten Speicherort;
   (5) Messen, mit dem Prozessor, von Restverschiebungswerten an den gemeinsamen Bildpunktsammlungen, wobei sich jeder Restverschiebungswert auf einen entsprechenden räumlichen Ort bezieht; und
   (6) Invertieren, mit dem Prozessor, der Restverschiebungswerte, um ein Schallgeschwindigkeitsbild zu erstellen, und Senden des Schallgeschwindigkeitsbildes an eine Netzadresse über TCP/IP, einen Display-Port auf einem Host-Computer oder einen dritten Speicherort.

2. Verfahren nach Anspruch 1, wobei der Schritt (6) umfasst:

   Einstellen einer Anfangsschallgeschwindigkeit; und
   Berechnen, mit dem Prozessor, eines aktualisierten Schallgeschwindigkeitswertes an dem entsprechenden räumlichen Ort unter Verwendung der anfänglichen Schallgeschwindigkeit und des jeweiligen Restverschiebungswertes.

3. Verfahren nach Anspruch 2, wobei die Anfangsschallgeschwindigkeit 1.540 m/s beträgt.

4. Verfahren nach Anspruch 2, wobei der Schritt (6) ferner umfasst:
   Anwenden einer räumlichen Glättung, mit dem Prozessor, auf das Schallgeschwindigkeitsbild, um schnelle Schwankungen zu entfernen.

5. Verfahren zum Erhalten einer Poissonzahl, die Schritte umfassend:

   (1) Verteilen, mit einem Prozessor, einer Datenprobe eines Ultraschallstrahls, die mit einem Gerät gemessen wurde, das einen Ultraschall-Array-Wandler enthält, in einen Bildraum eines Subjekts entlang einer Impulsantwortkurve;
   (2) Sammeln, mit dem Prozessor, von Beiträgen der Datenprobe im Bildraum;
   (3) Summieren der Beiträge einer Vielzahl von Datenproben durch Wiederholen des Schritts (1) und des Schritts (2) für eine Vielzahl von Ultraschallstrahlen mit dem Prozessor, um eine Vielzahl von Teilbildern zu erzeugen, und Speichern der Teilbilder an einem ersten Speicherort;
   (4) Sortieren, mit dem Prozessor, der Teilbilder, um gemeinsame Bildpunktsammlungen an einer Vielzahl von räumlichen Orten zu bilden, und Speichern der gemeinsamen Bildpunktsammlungen an einem zweiten Speicherort;
   (5) Messen, mit dem Prozessor, von Restverschiebungswerten an den gemeinsamen Bildpunktsammlungen, wobei sich jeder Restverschiebungswert auf einen entsprechenden räumlichen Ort bezieht;
   (6) Anwenden, mit dem Prozessor, von Restverschiebungskorrekturen, um die gemeinsamen Bildpunktsammlungen zu glätten;
   (7) Messen, mit dem Prozessor, von Amplituden und Reflexionswinkeln an den gemeinsamen Bildpunktsammlungen, wobei sich jedes Paar von Amplitude und Reflexionswinkel auf einen entsprechenden räumlichen Ort bezieht; und
   (8) Invertieren, mit dem Prozessor, der Amplituden und Reflexionswinkel, um ein Bild der Poissonzahl zu erstellen, und Senden des Bildes der Poissonzahl an eine Netzadresse über TCP/IP, einen Display-Port auf einem Host-Computer oder einen dritten Speicherort.

**6.** Verfahren nach Anspruch 5, wobei der Schritt (8) umfasst:

Schätzen einer normalen Amplitude der einfallenden Reflexion; und
Berechnen, mit dem Prozessor, der Poissonzahl an dem entsprechenden räumlichen Ort unter Verwendung der normalen Amplitude der einfallenden Reflexion und der gemessenen Amplituden bei einer Vielzahl von Reflexionswinkeln.

**7.** Verfahren nach Anspruch 5, wobei der Schritt (8) umfasst:

Schätzen einer normalen Amplitude der einfallenden Reflexion; und
Berechnen, mit dem Prozessor, einer Stellvertretergröße für den Scherwellengeschwindigkeitskontrast an dem entsprechenden räumlichen Ort unter Verwendung der normalen Amplitude der einfallenden Reflexion und der gemessenen Amplituden bei einer Vielzahl von Reflexionswinkeln.

**Revendications**

**1.** Procédé d'obtention d'une image de vitesse du son, comprenant les étapes de :

(1) pulvérisation, avec un processeur, d'un échantillon de données d'un faisceau ultrasonore, mesuré avec un appareil qui contient un transducteur à réseau ultrasonore, dans un espace image d'un sujet le long d'une courbe de réponse impulsionnelle ;
(2) collecte, avec le processeur, de contributions de l'échantillon de données dans l'espace image ;
(3) somme des contributions d'une pluralité d'échantillons de données en répétant l'étape (1) et l'étape (2) pour une pluralité de faisceaux ultrasonores, avec le processeur, pour générer une pluralité d'images partielles, et stockage des images partielles dans un premier emplacement de mémoire ;
(4) tri, avec le processeur, des images partielles pour former des ensembles de points d'image communs en une pluralité d'emplacements spatiaux, et stockage des ensembles de points d'image communs dans un second emplacement de mémoire ;
(5) mesure, avec le processeur, de valeurs de suppression résiduelle sur les ensembles de points d'image communs, chaque valeur de suppression résiduelle se rapportant à un emplacement spatial correspondant ; et
(6) inversion, avec le processeur, des valeurs résiduelles de suppression pour former une image de la vitesse du son, et envoi de l'image de la vitesse du son à une adresse réseau via TCP/IP, un port d'affichage sur un ordinateur hôte ou un troisième emplacement de mémoire.

**2.** Procédé selon la revendication 1, dans lequel l'étape (6) comprend :

le réglage d'une vitesse du son initiale ; et
le calcul, avec le processeur, d'une valeur de vitesse du son mise à jour à l'emplacement spatial correspondant à l'aide de la vitesse du son initiale et de chaque valeur de suppression résiduelle.

**3.** Procédé selon la revendication 2, dans lequel la vitesse du son initiale est de 1 540 m/s.

**4.** Procédé selon la revendication 2, dans lequel l'étape (6) comprend en outre :
l'application d'un lissage spatial, avec le processeur, à l'image de vitesse du son pour supprimer des variations rapides.

**5.** Procédé d'obtention d'une image à coefficient de Poisson, comprenant les étapes de :

(1) pulvérisation, avec un processeur, d'un échantillon de données d'un faisceau ultrasonore, mesuré avec un appareil qui contient un transducteur à réseau ultrasonore, dans un espace image d'un sujet le long d'une courbe de réponse impulsionnelle ;
(2) collecte, avec le processeur, de contributions de l'échantillon de données dans l'espace image ;
(3) somme des contributions d'une pluralité d'échantillons de données en répétant l'étape (1) et l'étape (2) pour une pluralité de faisceaux ultrasonores, avec le processeur, pour générer une pluralité d'images partielles, et stockage des images partielles dans un premier emplacement de mémoire ;

(4) tri, avec le processeur, des images partielles pour former des ensembles de points d'image communs en une pluralité d'emplacements spatiaux, et stockage des ensembles de points d'image communs dans un second emplacement de mémoire ;

(5) mesure, avec le processeur, de valeurs de suppression résiduelle sur les ensembles de points d'image communs, chaque valeur de suppression résiduelle se rapportant à un emplacement spatial correspondant ; et

(6) application, avec le processeur, de corrections de suppression résiduelle pour aplatir les ensembles de points d'image communs ;

(7) mesure, avec le processeur, d'amplitudes et d'angles de réflexion sur les ensembles de points d'image communs, chaque paire d'une amplitude et d'un angle de réflexion se rapportant à un emplacement spatial correspondant ; et

(8) inversion, avec le processeur, des amplitudes et des angles de réflexion pour former une image du coefficient de Poisson, et envoi de l'image du coefficient de Poisson à une adresse réseau via TCP/IP, un port d'affichage sur un ordinateur hôte ou un troisième emplacement de mémoire.

6. Procédé selon la revendication 5, dans lequel l'étape (8) comprend :

l'estimation d'une amplitude de réflexion incidente normale ; et

le calcul, avec le processeur, d'un proxy du coefficient de poisson à l'emplacement spatial correspondant en utilisant l'amplitude de réflexion incidente normale et les amplitudes mesurées à une pluralité d'angles de réflexion.

7. Procédé selon la revendication 5, dans lequel l'étape (8) comprend :

l'estimation d'une amplitude de réflexion incidente normale ; et

le calcul, avec le processeur, d'un proxy du contraste de vitesse des ondes de cisaillement à l'emplacement spatial correspondant à l'aide de l'amplitude de réflexion incidente normale et des amplitudes mesurées à une pluralité d'angles de réflexion.

*FIG. 1*

*FIG. 2*

*FIG. 3*

*FIG. 4*

*FIG. 5*

*FIG. 6*

*FIG. 7*

*FIG. 8*

| Data Processing Flow | Main Architecture of the Apparatus |
|---|---|
| Spray data samples of ultrasound beams<br><br>Collect image contributions of all data samples<br><br>Store partial images in a first memory location<br><br>Sort partial images into common image point gathers<br><br>Store common image point gathers in a second memory location<br><br>Measure residual moveout values<br><br>Invert for sound speed values<br><br>Send sound speed images to devices or a third memory location (for further processing) | |

*FIG. 9*

| Data Processing Flow | Main Architecture of the Apparatus |
|---|---|
| Spray data samples of ultrasound beams<br><br>Collect image contributions of all data samples<br><br>Store partial images in a first memory location<br><br>Sort partial images into common image point gathers<br><br>Store common image point gathers in a second memory location<br><br>Measure residual moveout values and apply residual moveout corrections<br><br>Measure reflection amplitudes and reflection angles on common image point gathers<br><br>Invert for Poisson's ratio values (proxy)<br><br>Send Poisson's ratio images to devices or a third memory location (for further processing) | Ultrasound Array Transducer → GPU Processor(s) → Host Computer<br><br>GPU Processor(s) ↔ First Memory location<br>GPU Processor(s) ↔ Second Memory location<br>Host Computer ↔ Third Memory location<br>Host Computer → Display Port<br>Host Computer → Network Connection |

Data Processing Flow

Main Architecture of the Apparatus

*FIG. 10*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63229246
- US 2021177380 A1
- US 63184174
- US 2024210560 A1
- US 63197932
- US 2024206854 A1
- US 20090112095 A1
- US 8002705 B

### Non-patent literature cited in the description

- **ISLAM MD. TAUHIDUL et al.** Non-invasive imaging of Young's modulus and Poisson's ratio in cancers in vivo. *SCIENTIFIC REPORTS*, 29 April 2020, vol. 10 (1), ISSN 2045-2322
- **RICHARD S. C. COBBOLD**. Foundations of Biomedical Ultrasound. Oxford University Press, 2007, 1416-1428
- **F. W. KREMKAU**. Diagnostic Ultrasound: Principles and Instruments. Saunders/ Elsevier, 2006
- **J. OPHIR** ; **S. K. ALAM** ; **B. S. GARRA** ; **F. KALLEL** ; **E. E. KNONFGOU** ; **T. KROUSKO** ; **C. R. B. BERRITT**. Elastography: Imaging the Elastic Properties of Soft Tissues with Ultrasound. *J. Medical Ultrasonics*, 2002, vol. 29, 155-171
- **J. BERCOFF** ; **M. TANTER** ; **T. M. NGUYEN** ; **J. M. CHASSOT** ; **M. FINK** ; **C. BOCCARA**. Supersonic Shear Imaging: A New Technique for Soft Tissue Elasticity Mapping. *IEEE Trans. On Ultrasonics, Ferroelectrics and Frequency Control*, 2004, vol. 51, 396-409
- **M. SAK** ; **N. DURIC** ; **P. LITTRUP** ; **L. BEY-KNIGHT** ; **H. ALI** ; **P. VALLIERES** ; **M. E. SHERMAN** ; **G. L. GIERACH**. Using Speed of Sound Imaging to Characterize Breast Density. *Ultrasound in Medicine & Biology*, 2017, vol. 43, 91-103
- **C. LI** ; **A. STEWART** ; **N. DURIC**. Multi-grid Tomographic Inversion for Breast Ultrasound Imaging. *Proceedings of SPIE*, 2012, vol. 8320, 1-9
- **J. NEBEKER** ; **T. R. NELSON**. Imaging of Sound Speed Using Reflection Ultrasound Tomography. *Journal of Ultrasound in Medicine*, 2012, vol. 31, 1389-1404
- **MICHA FEIGIN** ; **D. FREEDMAN** ; **B. W. ANTHONY**. Deep Learning Framework for Single-Sided Sound Speed Inversion in Medical Ultrasound. *IEEE Transactions on Biomedical Engineering*, vol. 67, 1142-1150
- **J. WISKIN** ; **B. MALIK** ; **D. BORUP** ; **N. PIRSHA-FIERY** ; **J. KLOCK**. Full Wave 3D Inverse Scattering Transmission Ultrasound Tomography in the Presence of High Contrast. *Nature*, 2020, https://doi.org/10.1038/s41498-020-76754-3
- **L. GUASCH** ; **O. CALDERON-AGUDO** ; **M. X. TANG** ; **P. NACHEV** ; **M. WARNER**. Full-waveform Inversion Imaging of the Human Brain. *Nature*, 2020, https://doi.org/10.1038/s41746-020-0240-8
- **R. E. DAIGLE**. *Ultrasound Imaging System with Pixel Oriented Processing*, 2009
- **R. ZEMP** ; **M. F. INSANA**. Imaging with Unfocused Regions of Focused Ultrasound Beams. *J. Acoust. So. Amer.*, 2007, vol. 121, 1491-1498
- **N. Q. NGUYEN** ; **RICHARD Q. PRAGER**. High-resolution Ultrasound Imaging with Unified Pixel-Based Beamforming. *IEEE Transactions on Medical Imaging*, 2016, vol. 35, 98-108
- **O. M. H. RINDAL**. Software Beamforming in Medical Ultrasound Imaging - a Blessing and a Curse. *Ph.D. Thesis, University of Oslo*, 2019
- **O. M. H. RINDAL** ; **A. RODRIGUEZ-MOLARES** ; **A. AUSTENG**. A Simple, Artifact-free, Virtual Source Model. *IEEE International Ultrasonics Symposium, IUS 1-4*, 2018, https://doi.org/10.1109/ultsym.2018.8579944
- **D. J. NAPOLITANO** ; **B. D. DEBUSSCHERE** ; **G. W. MCLAUGHLIN** ; **L. Y. MO** ; **C. H. CHOU** ; **T. L. JI** ; **R. W. STEINS**. *Continuous Transmit Focusing Method and Apparatus for Ultrasound Imaging Systems*, 2011
- **C. PENG** ; **J. TANG**. *Acquisition and Processing of V-Wave Ultrasound Data Using a Linear or Curved Array Transducer*, 2021
- **C. PENG** ; **J. TANG**. *Imaging Tissues and Organs Behind Bones Using an Ultrasound Array Transducer*, 2021
- **C. H. DIX**. Seismic Velocities from Surface Measurements. *Geophysics*, 1955, vol. 20, 68-86
- **Z. KOREN** ; **I. RAVVE**. Constrained Dix Inversion. *Geophysics*, 2006, vol. 71, R113-R130

- **K, AKI** ; **P. G. RICHARDS**. Quantitative Seismology: Theory and Methods. Freeman and Co, 1980
- **O. YILMAZ**. Seismic Data Analysis: Processing, Inversion and Interpretation of Seismic Data. Society of Exploration Geophysicists, 2011
- **R. BORTFELD**. Approximation to the Reflection and Transmission Coefficients of Plane Longitudinal and Transverse Waves. *Geophysical Prospecting*, 1961, vol. 9, 485-503
- **J. P. CASTAGNA** ; **H. W. SWAN** ; **D. J. FOSTER**. Framework for AVO Gradient and Intercept Interpretation. *Geophysics*, 1998, vol. 63, 948-956